# EUROPÄISCHE PATENTSCHRIFT

(11) **EP 2 651 802 B1**
(45) Veröffentlichungstag und Bekanntmachung des Hinweises auf die Patenterteilung: **25.02.2015**
(21) Anmeldenummer: 11784919.0
(22) Anmeldetag: 11.11.2011
(51) Int. Cl.: B65H 63/06, G01N 33/36

(54) **DIAGNOSEVERFAHREN FÜR EINE TEXTILE MESSVORRICHTUNG**
DIAGNOSTIC METHOD FOR A TEXTILE MEASURING APPARATUS
PROCÉDÉ DE DIAGNOSTIC POUR UN DISPOSITIF DE MESURE DE PRODUITS TEXTILES

(30) Priorität: 13.12.2010 CH 20792010
(43) Veröffentlichungstag der Anmeldung: 23.10.2013
(73) Patentinhaber: Uster Technologies AG, 8610 Uster (CH)
(72) Erfinder: KELLER, Beat, CH-8046 Zürich (CH); SCHMID, Peter, CH-8050 Zürich (CH); DE VRIES, Loris, CH-8616 Riedikon (CH)
(74) Vertreter: Pliska, Pavel
(86) Internationale Anmeldenummer: PCT/CH2011/000271
(87) Internationale Veröffentlichungsnummer: WO 2012/079181

(56) Entgegenhaltungen:
- WO-A1-2008/154756
- WO-A2-03/085179
- DE-A1- 3 137 315
- DE-A1- 19 535 177
- DE-A1-102007 062 601
- DE-A1-102008 000 610
- US-A- 5 748 481

## Beschreibung

### FACHGEBIET

Die vorliegende Erfindung liegt auf dem Gebiet der textilen Materialprüfung. Sie betrifft ein Diagnoseverfahren für eine Vorrichtung zur Messung mindestens eines Parameters eines textilen Prüfgutes, gemäss dem Oberbegriff des ersten Patentanspruchs. Sie betrifft ferner ein Verfahren zum Betrieb einer Vorrichtung zur Messung mindestens eines Parameters eines textilen Prüfgutes und eine Vorrichtung zur Messung mindestens eines Parameters eines textilen Prüfgutes, gemäss den Oberbegriffen weiterer unabhängiger Patentansprüche. Die Erfindung kann bspw. in Garnreinigern auf Spinn- oder Spulmaschinen eingesetzt werden.

### STAND DER TECHNIK

Es ist eine Vielzahl verschiedenartiger Vorrichtungen und Verfahren zur Untersuchung von Textilmaterialien bekannt. Sie lassen sich nach ihrer Anwendung in die beiden Klassen Laborprüfung (offline) und Prüfung während des Produktionsprozesses (online) einteilen. In den Textilprüfvorrichtungen kommen verschiedene Sensorprinzipien zur Anwendung; der Einsatz eines bestimmten Sensorprinzips hängt unter anderem davon ab, welche Eigenschaft optimal detektiert werden soll. Häufig verwendete Sensorprinzipien, besonders in der Garnprüfung, sind das kapazitive und das optische. Beim letzteren wird das Garn von einer Lichtquelle beleuchtet, und mit dem Garn wechselwirkendes Licht wird von einem Lichtdetektor detektiert. Daraus lässt sich bspw. die Garndicke oder das Vorhandensein von Fremdstoffen bestimmen.

Ein Problem bei der Untersuchung von Textilmaterialien ist die Verschmutzung des Sensors. Die Schmutzpartikel können vom Textilmaterial selbst stammen, wie z. B. Honigtau, Wachs oder Baumwollabrieb, und/oder von einer externen Schmutzquelle, wie z. B. Russpartikel oder schmutziges Wasser aus einem Nassspleisser. Die Verschmutzung verfälscht die Messresultate bei vielen Sensortypen. Am meisten jedoch sind die optischen Sensoren davon betroffen. Schmutzablagerungen auf optischen Komponenten im Lichtpfad, z. B. auf einen Messschlitz begrenzenden Fenstern, absorbieren das Messlicht. Die Empfindlichkeit des Sensors nimmt dadurch ab. Durch die Verschmutzung wird das Messergebnis verfälscht, was zu Fehlinterpretationen führen kann.

Der Stand der Technik schlägt verschiedene Massnahmen zur Verminderung des Verschmutzungsproblems vor. Eine solche Massnahme ist die Reinigung der Sensorflächen. Die DE-10'2008'000'610 A1 stellt fest, dass keine gesicherten Voraussagen für einen kritischen Verschmutzungszustand getroffen werden können. Deshalb müsse in vorbeugenden Zeitabständen ein Reinigungsvorgang der Sensorflächen veranlasst werden. Das Verfahren gemäss der US-5,748,481 A vergleicht die Messwerte des betreffenden Sensors mit denjenigen anderer Sensoren und gibt ein Warnsignal aus, wenn eine Abweichung festgestellt wird.

Eine weitere, aus dem Stand der Technik bekannte Massnahme besteht darin, die Verschmutzung automatisch zu kompensieren. Zu diesem Zweck offenbart die US-3,309,754 A eine Sensorschaltung, welche Intensitätsänderungen kompensiert, die eine vorbestimmte Zeitdauer überschreiten. Somit werden durch Verschmutzung des Sensors verursachte Intensitätsänderungen kompensiert, wohingegen Garnfehler im Sensorsignal erkennbar sind. Gemäss der EP-0'572'592 B1 wird das Sensorsignal dazu verwendet, die Intensität einer Sensorlichtquelle zu regeln. Die Regelung erfolgt so langsam, dass Garnfehler im Sensorsignal erkennbar sind, hingegen durch Verschmutzung des Sensors verursachte Intensitätsänderungen keinen Einfluss auf das Sensorsignal haben. Die GB-1'428'757 A schlägt vor, abwechslungsweise vom Textilmaterial beeinflusstes und vom Textilmaterial unbeeinflusstes Licht auf denselben Detektor einfallen zu lassen und die Verstärkung des Detektorsignals so zu regeln, dass die vom Textilmaterial unbeeinflussten Signalanteile auf einem konstanten Niveau verbleiben.

Nebst der Verschmutzung gibt es auch weitere Ursachen für nicht ordnungsgemässe Zustände von Vorrichtung zur Untersuchung von Textilmaterialien. Eine solche weitere Ursache ist z. B. die Alterung von Komponenten der Vorrichtung, bspw. einer Lichtquelle in einer optischen Sensoreinheit. Eine andere Ursache kann ein Verschleiss oder eine Abnutzung von Komponenten der Vorrichtung sein, bspw. eine Abrasion einer einen Messschlitz begrenzenden optischen Fläche in einer optischen Sensoreinheit.

### DARSTELLUNG DER ERFINDUNG

Es ist eine Aufgabe der vorliegenden Erfindung, ein Diagnoseverfahren für eine Vorrichtung zur Messung mindestens eines Parameters eines textilen Prüfgutes anzugeben, mittels dessen ein nicht ordnungsgemässer Zustand der Vorrichtung, bspw. einen Verschmutzungsgrad, einen Alterungsgrad oder einen Abnutzungsgrad der Vorrichtung, automatisch und zuverlässig erkannt wird. Somit sollen sich periodische, vorbeugende Reinigungen der Vorrichtung erübrigen. Hingegen soll bei einem stark von der Norm abweichenden Zustand, bspw. bei einer starken Verschmutzung, eine Warnung ausgegeben werden, so dass die Vorrichtung gezielt gewartet werden kann. Eine derartige Wartung kann z. B. eine Reinigung von stark verschmutzten Teilen der Vorrichtung und/oder einen Austausch von Teilen der Vorrichtung beinhalten.

Ferner soll ein Verfahren zum Betrieb einer Vorrichtung zur Messung mindestens eines Parameters eines textilen Prüfgutes und eine solche Vorrichtung angegeben werden, in welchen das erfindungsgemässe Diagnoseverfahren eingesetzt wird.

Diese und andere Aufgaben werden durch das erfindungsgemässe Diagnoseverfahren, das erfindungsgemässe Betriebsverfahren und die erfindungsgemässe Vorrichtung, wie sie in den unabhängigen Patentansprüchen definiert sind, gelöst. Vorteilhafte Ausführungsformen sind in den abhängigen Patentansprüchen angegeben.

Im erfindungsgemässen Diagnoseverfahren für eine Vorrichtung zur Messung mindestens eines Parameters eines textilen Prüfgutes wird der mindestens eine Parameter des textilen Prüfgutes mit der Vorrichtung gemessen. Ein Resultat der Messung wird mit einem Sollwert verglichen, der für das ausgemessene Prüfgut charakteristisch ist und von Messungen anderer derartiger Vorrichtungen unabhängig ist. Aus dem Vergleich wird auf einen Zustand der Vorrichtung geschlossen.

Der Sollwert ist insofern von Messungen anderer derartiger Vorrichtungen unabhängig, als dass er nicht z. B. durch Mittelwertbildung entsprechender Signale von einer Vielzahl identischer Vorrichtungen gebildet wird, wie dies aus der US-5,748,481 A bekannt ist. Dies hat erstens den Vorteil, dass das erfindungsgemässe Verfahren somit auch für eine allein stehende Vorrichtung funktioniert, die nicht auf den gleichzeitigen Betrieb weiterer, identischer Schwesternvorrichtungen angewiesen ist. Zweitens ist das erfindungsgemässe Verfahren zuverlässiger als dasjenige gemäss dem Stand der Technik, denn das letztere versagt, wenn sich alle Vorrichtungen in einem gleichermassen nicht ordnungsgemässen Zustand befinden.

In einer bevorzugten Ausführungsform ist der Zustand, auf den aus dem Vergleich geschlossen wird, ein Verschmutzungsgrad, ein Alterungsgrad oder ein Abnutzungsgrad der Vorrichtung.

Vorzugsweise wird ein Warnsignal ausgegeben, wenn eine Differenz zwischen dem Sollwert und dem Resultat der Messung einen vorgegebenen Schwellenwert überschreitet. Der Sollwert kann ein vorgegebener Wert sein oder aus mindestens einem vorgegebenen Wert ermittelt werden. Das textile Prüfgut ist z. B. ein längliches, im Wesentlichen zylinderförmiges textiles Gebilde wie Garn oder Faserband, und der Sollwert wird aus einem vorgegebenen Wert, der mit dem Durchmesser des textilen Gebildes zusammenhängt, bspw. aus einer Garnnummer oder einer Bandnummer, ermittelt. In einer bevorzugten Ausführungsform ist die Vorrichtung ein Garnreiniger, insbesondere ein optischer Garnreiniger, und das textile Prüfgut ist ein Garn.

Die Erfindung betrifft auch ein Verfahren zum Betrieb einer Vorrichtung zur Messung mindestens eines Parameters eines textilen Prüfgutes. Dabei wird ein Diagnoseverfahren für die Vorrichtung durchgeführt und eine Messung des mindestens einen Parameters des textilen Prüfgutes vorgenommen. Das Diagnoseverfahren ist das oben beschriebene Diagnoseverfahren.

Nach dem Diagnoseverfahren und vor der Messung wird vorteilhafterweise ein Eingriff an der Vorrichtung durchgeführt, wenn das Diagnoseverfahren einen nicht ordnungsgemässen Zustand ergibt. Der Eingriff besteht z. B. in einer Reinigung oder einem Austausch von Teilen der Vorrichtung. Zusätzlich oder alternativ zur Reinigung kann nach dem Diagnoseverfahren die Vorrichtung auf den im Diagnoseverfahren verwendeten Sollwert justiert und diese Justierung in der nachfolgenden Messung verwendet werden. Die Justierung wird dann in allen nachfolgenden Messungen bis zur nächsten Justierung verwendet.

Die erfindungsgemässe Vorrichtung zur Messung mindestens eines Parameters eines textilen Prüfgutes beinhaltet einen Sensor für den mindestens einen Parameter des Prüfgutes und eine Auswerteeinheit zur Auswertung von Ausgangssignalen des Sensors. Die Auswerteeinheit ist zur Ausführung des oben beschriebenen Diagnoseverfahrens und des oben beschriebenen Betriebsverfahrens eingerichtet ist.

In einer bevorzugten Ausführungsform ist der Sensor ein kapazitiver oder ein optischer Sensor. Die Vorrichtung ist vorzugsweise ein Garnreiniger, insbesondere ein optischer Garnreiniger. Sie kann eine Eingabeeinheit zur Eingabe eines vorgegebenen Sollwertes für den mindestens einen Parameter aufweisen.

### AUFZÄHLUNG DER ZEICHNUNGEN

Nachfolgend wird eine bevorzugte Ausführungsform der Erfindung anhand der Zeichnungen detailliert erläutert.
- Figur 1: zeigt einen Sensor der erfindungsgemässen Vorrichtung.
- Figur 2: zeigt ein Flussdiagramm einer Ausführungsform des erfindungsgemässen Verfahrens.
- Figur 3: zeigt den Vergleich eines Messsignals mit einem Sollwert (a) bei geringer und (b) bei starker Verschmutzung.

### AUSFÜHRUNG DER ERFINDUNG

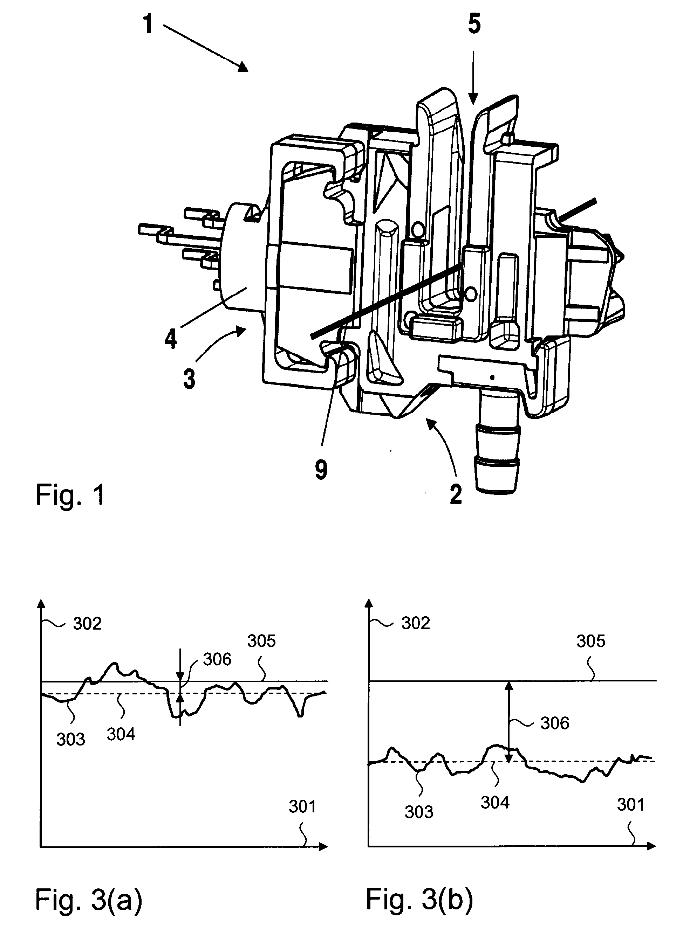

**Figur 1** zeigt eine optische Sensoreinheit 1, wie sie in einer erfindungsgemässen Vorrichtung eingebaut sein kann. Die Sensoreinheit 1 beinhaltet einen Messzellenkörper 2 mit mindestens einem Lichtdetektor und ein Beleuchtungselement 3 mit einer Lichtquelle, bspw. einer Leuchtdiode, die in einem Halteelement 4 gehalten sein kann. Der Messzellenkörper 2 ist ein komplexes Gebilde mit einem Messschlitz 5, durch welchen ein Prüfgut 9, bspw. ein Garn, in seiner Längsrichtung bewegt werden kann. Die Lichtquelle sendet Licht in den Messschlitz 5, wo es mit dem Prüfgut 9 wechselwirkt. Ein Teil des Lichtes wird vom mindestens einen Lichtdetektor detektiert. Die Ausgangssignale der Sensoreinheit 1 werden verstärkt und in einer (nicht dargestellten) Auswerteeinheit ausgewertet. So wird mindestens ein Parameter des Prüfgutes 9, z. B. der Durchmesser und/oder der Fremdstoffgehalt von Garn, gemessen. Die Auswertung kann ferner eine Bewertung der Qualität des ausgemessenen Prüfgutabschnitts beinhalten, bei welcher festgestellt wird, ob der mindestens eine gemessene Parameter ein vorgegebenes Qualitätskriterium erfüllt. Das Qualitätskriterium kann z. B. eine Reinigungsgrenze für Dickstellen oder für Fremdstoffe sein.

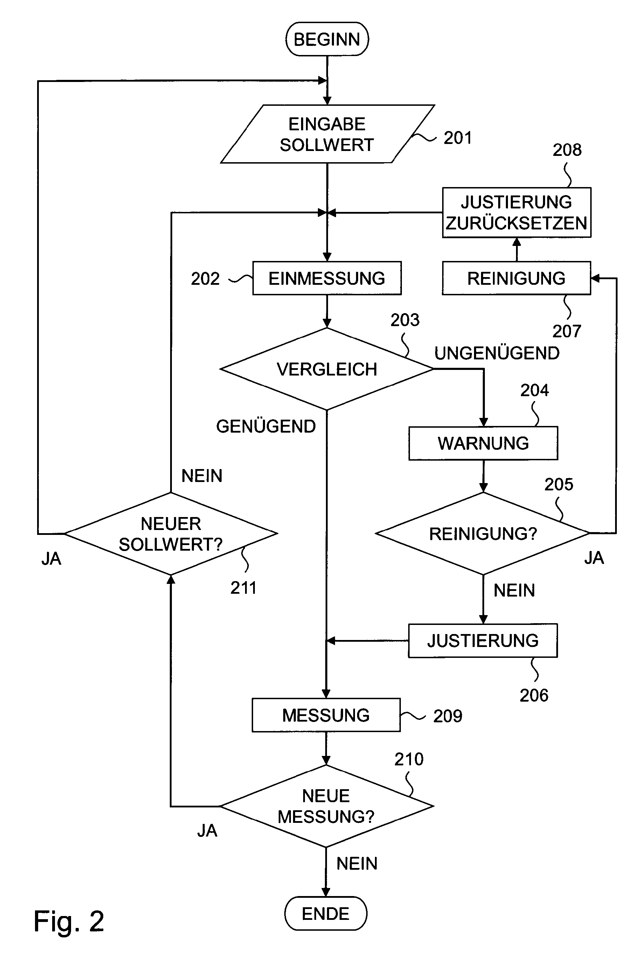

Da Flussdiagramm von **Figur 2** veranschaulicht eine Ausführungsform des erfindungsgemässen Diagnoseverfahrens. Es wird ein für das Prüfgut 9 charakteristischer Sollwert vorgegeben 201. Bei einer optischen Gammessvorrichtung kann dies z. B. eine vorbekannte Garnnummer des auszumessenden Garns 9 sein, aus der sich bei bekannter oder geschätzter Dichte der Garndurchmesser berechnen lässt. In einem Einmessschritt 202 wird ein Abschnitt des Prüfgutes 9 von der Sensoreinheit 1 ausgemessen. Dabei tastet die Sensoreinheit 1 das Prüfgut 9 entlang seiner Länge ab und misst mindestens einen Parameter des Prüfgutes 9. Der für die Einmessung 202 verwendete Prüfgutabschnitt sollte mindestens so lang sein, dass eine statistisch genügend repräsentative Probe des Prüfgutes 9 ausgemessen wird. Das heisst, dass sich eventuelle Fehlstellen im Prüfgut 9 nur unwesentlich auf das Resultat der Einmessung 202 auswirken dürfen. Für ein typisches Garn 9 sollten zu diesem Zweck einige Meter, z. B. 10 m, genügen.

Anschliessend an die Einmessung 202 wird ein Resultat der Einmessung 202 mit dem vorgegebenen Sollwert verglichen 203. Der Vergleich 203 ist anhand von **Figur 3** illustriert. Die Figur zeigt für zwei verschiedene Verschmutzungszustände jeweils ein Diagramm, in dem in der Einmessung erhaltene Messwerte eines Parameters 302 des Prüfgutes 9 gegenüber der Zeit 301 oder der Position auf dem Prüfgut 9 als Messkurve 303 dargestellt sind. Die Messkurve 303 ist hier als kontinuierliche Linie dargestellt, sie kann aber ebenso gut aus einer dichten Abfolge von diskreten Messpunkten zusammengesetzt sein. Als Resultat der Einmessung 202 kann z. B. ein Mittelwert aller entlang des Prüfgutabschnittes gemessenen Messwerte verwendet werden. Ein solcher Mittelwert ist als horizontal verlaufende, gestrichelte Gerade 304 eingezeichnet. Der vorgegebene Sollwert ist als horizontal verlaufende, ausgezogene Gerade 305 dargestellt. **Figur 3(a)** zeigt einen Zustand mit wenig verschmutzten optischen Oberflächen der Sensoreinheit 1. In diesem Zustand weicht der Mittelwert 304 der Einmessung 202 nur wenig vom Sollwert 305 ab, d. h. eine entsprechende Differenz 306 ist klein. Im Gegensatz dazu zeigt **Figur 3(b)** einen Zustand mit starker Verschmutzung. Hier ergibt sich eine grosse Differenz 306 zwischen dem Sollwert 305 und dem Mittelwert 304.

Im erfindungsgemässen Diagnoseverfahren, wie es in **Figur 2** dargestellt ist, wird nun das Resultat der Einmessung 202 mit dem vorgegebenen Sollwert 305 verglichen 203. Der Vergleich 203 kann z. B. durch Bildung der Differenz 306 zwischen dem Sollwert 305 und dem Messresultat 304 erfolgen. Liegt die Differenz 306 unterhalb eines vorgegebenen Schwellenwertes, wie in Figur 3(a), so ergibt der Vergleich 203 ein genügendes Resultat, d. h. die Sensoreinheit 1 ist nicht oder nur mässig verschmutzt. In diesem Fall kann eine eigentliche Messung 209 des Prüfgutes 9 erfolgen. Wie schon anlässlich der Einmessung 202 beschrieben, tastet bei der Messung 209 die Sensoreinheit 1 das Prüfgut 9 entlang seiner Länge ab und misst mindestens einen Parameter des Prüfgutes 9. Derartige Messungen 209 an einem textilen Prüfgut 9 sind aus dem Stand der Technik bestens bekannt. Nach Abschluss der Messung 209 kann eine neue Messung durchgeführt werden 210. Erfolgt diese an einem Prüfgut 9 desselben Typs (z. B. derselben Garnnummer) wie die vorhergehende Messung 209, so braucht der Sollwert 305 nicht verändert zu werden. Soll hingegen ein Prüfgut ausgemessen werden, das sich in wesentlichen Merkmalen vom vorhergehenden unterscheidet, so ist es von Vorteil, einen dem neuen Prüfgut entsprechenden neuen Sollwert einzugeben.

Wenn die Differenz 306 zwischen dem Sollwert 305 und dem Messresultat 304 den Schwellenwert überschreitet, so ergibt der Vergleich 203 ein ungenügendes Resultat, das auf eine starke Verschmutzung der Sensoreinheit 1 hinweist. Diesfalls wird automatisch eine entsprechende Verschmutzungswarnung ausgegeben 204. Die Warnung kann ein optisches und/oder akustisches Signal sein oder auf andere Art erfolgen. Die Sensoreinheit 1 kann daraufhin gereinigt werden oder nicht 205. Die Reinigung 207 erfolgt manuell durch eine Bedienungsperson oder automatisch durch eine entsprechende Einrichtung, bspw. einen Reinigungsroboter. Nach einer Reinigung 207 muss eine allfällige Justierung auf einen Anfangswert zurückgesetzt werden 208. Es sollte auch eine erneute Einmessung 202 erfolgen, um den nach der Reinigung 207 geringeren Verschmutzungsgrad zu berücksichtigen. Falls keine Reinigung erfolgt ist, kann die Vorrichtung zumindest justiert werden 206. Zu diesem Zweck wird für die nachfolgenden Messungen 209 ein Justieralgorithmus bereitgestellt, mittels dessen die Messwerte derart korrigiert werden, dass das Resultat der Messungen dem Sollwert 305 entspricht. Der Justieralgorithmus könnte z. B. eine Multiplikation der Messwerte mit einem Korrekturfaktor beinhalten, derart, dass der Mittelwert der korrigierten Einmesswerte dem Sollwert 305 entspricht. Ein solcher Korrekturfaktor ist gleich dem Verhältnis zwischen dem Sollwert 305 und dem bei der Einmessung 202 ermittelten Mittelwert 304.

Das obige Ausführungsbeispiel bezieht sich auf eine Verschmutzung der Sensoreinheit 1 (siehe Figur 1). Ein nicht ordnungsgemässer Zustand der erfindungsgemässen Vorrichtung kann aber auch andere Ursachen haben, bspw. eine Alterung der Leuchtdiode im Beleuchtungselement 3. Im letzteren Fall kann anstelle der Reinigung 207 (siehe Figur 2) ein Austausch der Leuchtdiode, des Beleuchtungselementes 3 oder der ganzen Sensoreinheit 1 erfolgen. Eine weitere Ursache für einen nicht ordnungsgemässen Zustand der Vorrichtung kann z. B. eine Abrasion eines lichtdurchlässigen Fensters sein, welches den Messschlitz 5 begrenzt. Diesfalls könnte ein angemessener Eingriff im Austausch des Messzellenkörpers 2 oder der ganzen Sensoreinheit 1 bestehen. Weitere Ursachen für nicht ordnungsgemässe Zustände der erfindungsgemässen Vorrichtung können durch analoge Eingriffe an der Vorrichtung behoben werden.

Selbstverständlich ist die vorliegende Erfindung nicht auf die oben diskutierten Ausführungsformen beschränkt. Bei Kenntnis der Erfindung wird der Fachmann weitere Varianten herleiten können, die auch zum Gegenstand der vorliegenden Erfindung gehören.

### BEZUGSZEICHENLISTE

- 1: Sensoreinheit
- 2: Messzellenkörper
- 3: Beleuchtungselement
- 4: Halteelement
- 5: Messschlitz
- 9: Prüfgut

- 201: Eingabe des Sollwertes
- 202: Einmessung
- 203: Vergleich zwischen einem Resultat der Einmessung und dem Sollwert
- 204: Warnung
- 205: Entscheidung über Reinigung
- 206: Justierung
- 207: Reinigung
- 208: Zurücksetzung der Justierung
- 209: Messung
- 210: Entscheidung über neue Messung
- 211: Entscheidung über neuen Sollwert

- 301: Achse für Zeit oder Position auf dem Prüfgut
- 302: Achse für Messwerte eines Parameters
- 303: Messkurve
- 304: Mittelwert der Messwerte
- 305: Sollwert
- 306: Differenz zwischen Sollwert und Messresultat

## Patentansprüche

1. Diagnoseverfahren für eine Vorrichtung zur Messung mindestens eines Parameters (302) eines textilen Prüfgutes (9), wobei
der mindestens eine Parameter (302) des textilen Prüfgutes (9) mit der Vorrichtung gemessen wird (202),
ein Resultat (304) der Messung (202) mit einem Sollwert (305) verglichen wird (203), der für das ausgemessene Prüfgut (9) charakteristisch ist und von Messungen anderer derartiger Vorrichtungen unabhängig ist, und
aus dem Vergleich (203) auf einen Zustand der Vorrichtung geschlossen wird.

2. Diagnoseverfahren nach Anspruch 1, wobei der Zustand, auf den aus dem Vergleich (203) geschlossen wird, ein Verschmutzungsgrad, ein Alterungsgrad oder ein Abnutzungsgrad der Vorrichtung ist.

3. Diagnoseverfahren nach einem der vorangehenden Ansprüche, wobei ein Warnsignal (204) ausgegeben wird, wenn eine Differenz (306) zwischen dem Sollwert (305) und dem Resultat (304) der Messung (202) einen vorgegebenen Schwellenwert überschreitet.

4. Diagnoseverfahren nach einem der vorangehenden Ansprüche, wobei der Sollwert (305) ein vorgegebener Wert ist oder aus mindestens einem vorgegebenen Wert ermittelt wird.

5. Diagnoseverfahren nach einem der vorangehenden Ansprüche, wobei das textile Prüfgut (9) ein längliches, im Wesentlichen zylinderförmiges textiles Gebilde wie Garn oder Faserband ist und der Sollwert (305) aus einem vorgegebenen Wert, der mit dem Durchmesser des textilen Gebildes zusammenhängt, bspw. aus einer Garnnummer oder einer Bandnummer, ermittelt wird.

6. Diagnoseverfahren nach einem der vorangehenden Ansprüche, wobei die Vorrichtung ein Garnreiniger, insbesondere ein optischer Garnreiniger, und das textile Prüfgut (9) ein Garn ist.

7. Verfahren zum Betrieb einer Vorrichtung zur Messung mindestens eines Parameters (302) eines textilen Prüfgutes (9), wobei
ein Diagnoseverfahren für die Vorrichtung durchgeführt wird und
eine Messung (209) des mindestens einen Parameters (302) des textilen Prüfgutes (9) vorgenommen wird,
**dadurch gekennzeichnet,**
**dass** das Diagnoseverfahren ein Diagnoseverfahren nach einem der vorangehenden Ansprüche ist.

8. Verfahren nach Anspruch 7, wobei nach dem Diagnoseverfahren und vor der Messung ein Eingriff (207) an der Vorrichtung durchgeführt wird, wenn das Diagnoseverfahren einen nicht ordnungsgemässen Zustand ergibt.

9. Verfahren nach Anspruch 8, wobei der Eingriff (207) in einer Reinigung oder in einem Austausch von Teilen der Vorrichtung besteht.

10. Verfahren nach einem der Ansprüche 7-9, wobei nach dem Diagnoseverfahren die Vorrichtung auf den im Diagnoseverfahren verwendeten Sollwert (305) justiert wird (206) und diese Justierung (206) in der nachfolgenden Messung (209) verwendet wird.

11. Verfahren nach Anspruch 10, wobei die Justierung (206) in allen nachfolgenden Messungen (209) bis zur nächsten Justierung verwendet wird.

12. Vorrichtung zur Messung mindestens eines Parameters (302) eines textilen Prüfgutes (9), beinhaltend
einen Sensor für den mindestens einen Parameter (302) des Prüfgutes (9) und
eine Auswerteeinheit zur Auswertung von Ausgangssignalen des Sensors,
**dadurch gekennzeichnet,**
**dass** die Auswerteeinheit zur Ausführung des Diagnoseverfahrens nach einem der Ansprüche 1-6 und des Betriebsverfahrens nach einem der Ansprüche 7-11 eingerichtet ist.

13. Vorrichtung nach Anspruch 12, wobei der Sensor ein kapazitiver oder ein optischer Sensor ist.

14. Vorrichtung nach Anspruch 12 oder 13, wobei die Vorrichtung ein Garnreiniger, insbesondere ein optischer Garnreiniger, ist.

15. Vorrichtung nach einem der Ansprüche 12-14, wobei die Vorrichtung eine Eingabeeinheit zur Eingabe eines vorgegebenen Sollwertes (305) für den mindestens einen Parameter (302) aufweist.

## Claims

1. A diagnostic method for an apparatus for measuring at least one parameter (302) of a textile sample (9), wherein
the at least one parameter (302) of the textile sample (9) is measured (202) using the apparatus,
a result (304) of the measurement (202) is compared (203) to a desired value (305), which is characteristic for the measured sample (9) and is independent of measurements of other such apparatuses, and
a state of the apparatus is concluded from the comparison (203).

2. The diagnostic method according to claim 1, wherein the state which is concluded from the comparison (203) is a degree of contamination, a degree of aging, or a degree of wear of the apparatus.

3. The diagnostic method according to one of the preceding claims, wherein a warning signal (204) is output if a difference (306) between the desired value (305) and the result (304) of the measurement (202) exceeds a predefined threshold value.

4. The diagnostic method according to one of the preceding claims, wherein the desired value (305) is a predefined value or is determined from at least one predefined value.

5. The diagnostic method according to one of the preceding claims, wherein the textile sample (9) is an oblong, substantially cylindrical textile formation such as yarn or sliver and the desired value (305) is determined from a predefined value which relates to the diameter of the textile formation, for example, from a yarn count or a sliver count.

6. The diagnostic method according to one of the preceding claims, wherein the apparatus is a yarn clearer, in particular an optical yarn clearer, and the textile sample (9) is a yarn.

7. A method for operating an apparatus for measuring at least one parameter (302) of
a textile sample (9), wherein
a diagnostic method for the apparatus is carried out and
a measurement (209) of the at least one parameter (302) of the textile sample (9) is performed,
**characterized in that**
the diagnostic method is a diagnostic method according to one of the preceding claims.

8. The method according to claim 7, wherein after the diagnostic method and before the measurement, an intervention (207) is carried out on the apparatus if the diagnostic method reveals an improper state.

9. The method according to claim 8, wherein the intervention (207) consists of cleaning or replacement of parts of the apparatus.

10. The method according to one of claims 7-9, wherein after the diagnostic method, the apparatus is adjusted (206) to the desired value (305) used in the diagnostic method and this adjustment (206) is used in the following measurement (209).

11. The method according to claim 10, wherein the adjustment (206) is used in all following measurements (209) until the next adjustment.

12. An apparatus for measuring at least one parameter (302) of a textile sample (9), containing
a sensor for the at least one parameter (302) of the sample (9) and
an analysis unit for analyzing output signals of the sensor,
**characterized in that**
the analysis unit is configured to execute a diagnostic method according to one of claims 1-6 and the operating method according to one of claims 7-11.

13. The apparatus according to claim 12, wherein the sensor is a capacitive sensor or an optical sensor.

14. The apparatus according to claim 12 or 13, wherein the apparatus is a yarn clearer, in particular an optical yarn clearer.

15. The apparatus according to one of claims 12-14, wherein the apparatus has an input unit for inputting a predefined desired value (305) for the at least one parameter (302).

## Revendications

1. Procédé de diagnostic pour un dispositif pour la mesure d'au moins un paramètre (302) d'une matière textile à contrôler (9), dans lequel
l'au moins un paramètre (302) de la matière textile à contrôler (9) est mesuré avec le dispositif (202),
un résultat (304) de la mesure (202) est comparé (203) avec une valeur de consigne (305) caractéristique de la matière à contrôler (9) mesurée et indépendante de mesures d'autres dispositifs de ce type, et
un état du dispositif est déduit de la comparaison (203).

2. Procédé de diagnostic selon la revendication 1, dans lequel l'état déduit de la comparaison (203) est un degré d'encrassement, un degré de vieillissement ou un degré d'usure du dispositif

3. Procédé de diagnostic selon l'une des revendications précédentes, dans lequel un signal d'avertissement (204) est émis quand une différence (306) entre la valeur de consigne (305) et le résultat (304) de la mesure (202) dépasse une valeur de seuil prédéfinie.

4. Procédé de diagnostic selon l'une des revendications précédentes, dans lequel la valeur de consigne (305) est une valeur prédéfinie ou est déterminée à partir d'au moins une valeur prédéfinie.

5. Procédé de diagnostic selon l'une des revendications précédentes, dans lequel la matière textile à contrôler (9) est une structure textile allongée et sensiblement cylindrique comme un fil ou un ruban de fibres et la valeur de consigne (305) est déterminée à partir d'une valeur prédéfinie corrélée au diamètre de la structure textile, par exemple à partir d'un numéro de fil ou d'un numéro de ruban.

6. Procédé de diagnostic selon l'une des revendications précédentes, dans lequel le dispositif est un nettoyeur de fils, en particulier un nettoyeur de fils optique, et la matière textile à contrôler (9) est un fil.

7. Procédé pour le pilotage d'un dispositif pour la mesure d'au moins un paramètre (302) d'une matière textile à contrôler (9), dans lequel
un procédé de diagnostic pour le dispositif est exécuté et
une mesure (209) de l'au moins un paramètre (302) de la matière textile à contrôler (9) est réalisée,
**caractérisé en ce que**
le procédé de diagnostic est un procédé de diagnostic selon l'une des revendications précédentes.

8. Procédé selon la revendication 7, dans lequel une intervention (207) sur le dispositif a lieu après le procédé de diagnostic et avant la mesure si le procédé de diagnostic signale un état non conforme.

9. Procédé selon la revendication 8, dans lequel l'intervention (207) consiste en un nettoyage ou un remplacement de pièces du dispositif.

10. Procédé selon l'une des revendications 7 à 9, dans lequel, après le procédé de diagnostic, le dispositif est réglé (206) à la valeur de consigne (305) utilisée dans le procédé de diagnostic et ce réglage (206) est utilisé dans la mesure (209) qui suit.

11. Procédé selon la revendication 10, dans lequel le réglage (206) est utilisé dans toutes les mesures (209) qui suivent jusqu'au réglage suivant.

12. Dispositif pour la mesure d'au moins un paramètre (302) d'une matière textile à contrôler (9), comprenant
un capteur pour l'au moins un paramètre (302) de la matière à contrôler (9) et
une unité d'analyse pour analyser des signaux de sortie du capteur,
**caractérisé en ce que**
l'unité d'analyse est conçue pour exécuter le procédé de diagnostic selon l'une des revendications 1 à 6 et le procédé de pilotage selon l'une des revendications 7 à 11.

13. Dispositif selon la revendication 12, dans lequel le capteur est un capteur capacitif ou un capteur optique.

14. Dispositif selon la revendication 12 ou 13, dans lequel le dispositif est un nettoyeur de fils, en particulier un nettoyeur de fils optique.

15. Dispositif selon l'une des revendications 12 à 14, dans lequel le dispositif comporte une unité de saisie pour la saisie d'une valeur de consigne (305) prédéfinie pour l'au moins un paramètre (302).
